(19) **Europäisches Patentamt**

**European Patent Office**

**Office européen des brevets**

(11) **EP 0 900 076 B1**

(12) ## EUROPEAN PATENT SPECIFICATION

(45) Date of publication and mention
of the grant of the patent:
**07.05.2003 Bulletin 2003/19**

(21) Application number: **97914286.6**

(22) Date of filing: **19.03.1997**

(51) Int Cl.[7]: **A61K 9/70**, A61M 37/00,
A61K 31/485

(86) International application number:
**PCT/EP97/01437**

(87) International publication number:
**WO 97/034587 (25.09.1997 Gazette 1997/41)**

(54) **TRANSDERMAL DEVICE**

TRANSDERMALE VORRICHTUNG

DISPOSITIF TRANSDERMIQUE

(84) Designated Contracting States:
**AT BE CH DE DK ES FI FR GB GR IE IT LI LU MC NL PT SE**

(30) Priority: **20.03.1996 GB 9605867**

(43) Date of publication of application:
**10.03.1999 Bulletin 1999/10**

(73) Proprietor: **Svedman, Pal**
**1137 Yens (CH)**

(72) Inventor: **Svedman, Pal**
**1137 Yens (CH)**

(74) Representative: **Baldock, Sharon Claire**
**BOULT WADE TENNANT,**
**Verulam Gardens**
**70 Gray's Inn Road**
**London WC1X 8BT (GB)**

(56) References cited:
**WO-A-92/11879        WO-A-95/15783**
**WO-A-95/30410        US-A- 3 964 482**

**Description**

The invention relates to transdermal delivery of a class of drug known as opiates or opioids. These include opioid alkaloids, of which the most well-known is morphine and synthetic piperidine analogues such as fentanyl and sufentanil. In particular the invention relates to a transdermal device which is suitable for continuous administration of an opiate/opioid analgesic via an area of skin from which the epidermis has been removed.

The opiates and opioids have powerful analgesic properties and morphine is the drug of choice for the treatment of severe post-operative pain and chronic pain associated with advanced cancer. It is usually administered orally or subcutaneously. Oral administration is highly effective and convenient but is not suitable for immediate post-operative care or in cases of terminal malignant disease when the drug absorption is impaired. Further, oral absorption is quite inefficient due to first pass metabolism through the liver.

As an alternative to the oral route morphine may be given subcutaneously, either by intermittent injection or continuous infusion. A disadvantage of the subcutaneous route is that the injection site is subject to local irritation and infection.

There are a number of other undesirable side-effects associated with administration of morphine and other opiates by the usual routes and these include changes in pupil size, reduced saliva production and various central nervous system effects such as nausea, fatigue, headache, feelings of heaviness and dysphoria/euphoria.

In recent years it has been discovered that a number of drugs can be efficiently administered through the skin. Transdermal administration has several advantages over the more conventional forms of drug delivery such as injection and oral ingestion. Firstly, transdermal devices can provide sustained and controlled release of the active agent over a prolonged period so that resulting blood levels remain constant. This is in contrast to administration by injection, for example, where surges of the agent occur in the bloodstream immediately after administration and then drop away rapidly until the next dose is given. Secondly, transdermal administration permits direct access to the bloodstream without passage through the gastrointestinal tract and liver as would be the case with oral administration. Finally, it is convenient and comfortable for patients because a small device or plaster can remain attached to the skin for a prolonged period without patient intervention. This is particularly advantageous, for example, for those patients needing post-operative care or who are terminally ill and cannot take drugs orally.

The possibility of administering opiate/opioid analgesics transdermally has been considered by Roy & Flynn in Pharmaceutical Research, Vol 6, No 10, 1989. In this study human cadaver skin was used to assess permeation *in vitro* of the six analgesics morphine, hydromorphone, codeine, fentanyl, sufentanil and meperidine. The work confirmed that the potential existed for transdermal administration of fentanyl and sufentanil but not for the opioid alkaloids because they were poorly permeable to the epidermis.

*In vivo* the epidermis provides a natural barrier against the ingress of foreign substances into the body and despite advances made in the field of transdermal administration, few drugs are able to permeate this barrier of their own accord. Permeation enhancers, which are able to increase the uptake of the active agent, are usually used. Electrochemical means to promote drug uptake have also been used with some success.

US Patent No. 3964482 describes a drug delivery device which comprises a plurality of projections for penetrating the stratum corneum of the epidermis, and a reservoir in immediate proximity with the projections for supplying a drug for percutaneous administration through the stratum corneum penetrated by the projections.

Another technique for circumventing the epidermal barrier has been developed by the present inventor which comprises administering a drug transdermally through a patch of skin which is de-epithelialized i.e. has a portion of the epidermis absent, whether or not deliberately removed.

A standardized de-epithelialized lesion of predetermined size can be made using devices such as those described in the Applicant's International Application Nos WO 92/11879 and WO 95/15783. These devices when attached to the skin apply suction to delaminate the epidermis from the dermis and so form a blister containing a clear blister fluid. The roof of the blister comprises the epidermis and can easily be removed leaving a standard sized de-epithelialized lesion where the dermis is exposed. Typical lesions are about 5 to 10 mm in diameter and about 200 µm to 1000 µm deep when made on the lower forearm, for example. These lesions are suitable for the application of a drug-loaded transdermal device and without the epidermal barrier the uptake of pharmaceutically active agents through the skin and into the bloodstream has been demonstrated to be much improved.

The Applicant's International Application No. WO 95/30410 describes a transdermal device suitable for delivery of a pharmaceutical to the systemic circulation through de-epithelialized skin.

The present inventor has applied this technique to transdermal administration of morphine as reported in Br. J. Clin Pharmac (1994) 37 571-576. In this study a 5 mm diameter de-epithelialized lesion was prepared using suction on the forearm of healthy volunteers. To this was applied an open plastic chamber into which 10 mg of morphine was injected. The chamber was removed after 24 hours and blood samples taken for up to 72 hours after morphine was first administered for measurement of morphine and its metabolites in the bloodstream. Non-analgesic effects such as change in pupil size and saliva production and CNS effects such as dysphoria/euphoria, fatigue, headache, nausea and heaviness, were also monitored. The results obtained were compared with subjects who had received an intravenous infusion of 10 mg morphine.

This study confirmed the feasibility of transdermal administration of morphine through de-epithelialized skin, since uptake was significantly improved. The morphine, in aqueous solution, does not restrict the rate of the drug delivery into the body. The epidermal barrier which normally prevents or pronouncedly restricts drug passage is absent and as a consequence drug will diffuse directly into the tissue from the reservoir. Under these conditions the small morphine molecule is expected to diffuse into the circulation without significant impediment. Given this background, the study showed that the rate of absorption exhibited first-order kinetics and the amount of drug in the reservoir was reduced by as much as 75 per cent (mean) by the end of the 24 hour delivery. It appeared to be clear that a further increase in dose beyond 10 mg morphine would lead to further increased morphine levels in the plasma at an early stage, i.e. a constant fraction of the dose would assumedly be absorbed per unit time by a free, passive diffusion process. The non-analgesic effects of morphine were much less pronounced after transdermal administration as compared with the intravenous route.

The inventor has now carried out a further study using a transdermal device containing significantly higher amounts of morphine than 10 mg and surprisingly found that the maximum plasma level of morphine, $C_{MAX,}$ is not substantially increased by the higher dose. Rather, a higher concentration is maintained over a much longer period as a result of increasing the total amount of drug. This can be explained by assuming that the absorption is markedly restricted by the small size of the erosion, and the constant delivery may be visualized as sand running through an hourglass. This was not to be expected from the previous study and makes transdermal administration of morphine through de-epithelialized skin a preferred option, particularly in the case of post-operative pain relief and terminal malignant disease. A transdermal device loaded with a higher dose of morphine or other opiate/opioid analgesic than could normally be administered by other routes can be applied to the lesion safely to maintain steady blood levels of the drug over a prolonged period.

Thus in accordance with the present invention there is provided a transdermal device suitable for continuous administration of an opiate/opioid analgesic for a period of about 24 to about 144 hours via an area of skin for which the epidermis has been removed which device comprises from 11 to 3000 mg of morphine or a salt thereof or from $\frac{11}{P}$ to $\frac{3000}{P}$ mg of another opiate/opioid analgesic wherein P is the level of analgesic effect of said other opiate/opioid analgesic relative to morphine.

Preferably, the device is suitable for continuous administration for a period of about 48 to about 144 hours.

Suitable opiate/opioid analgesics in addition to morphine which may be delivered transdermally by such a device are heroin, hydromorphone, ketobemidone, methadone, oxymorphone, levophanol, alfentanil, fentanyl, meperidine, sufentanil buprenorphine, pentazocine, nelburphine, butorphanol and the salts thereof.

P is the relative potency of any of the above compared to morphine. To determine a suitable weight of an opiate/opioid analgesic other than morphine for use in the device of the invention, the morphine dose should be divided by the P value for the chosen analgesic.

Potency values for the opiate/opioid analgesics listed above are set out in the Table 1 below. This table allows the appropriate quantity of opiate/opioid analgesic for the intended purpose to be calculated. The value of P for other analgesics not listed would be known to the skilled man.

TABLE 1

|  | P |
| --- | --- |
| Morphine | 1.0 |
| Heroin | 2.0 |
| Hydromorphone | 5.0-7.0 |
| Ketobemidone | 1.4 |
| Methadone | 1.0 |
| Oxymorphone | 10.0 |
| Levophanol | 5.0 |
| Alfentil | 10-30 |
| Fentanyl | 80-100 |
| Sufentanil | 500-800 |
| Meperidine | 8.0 |
| Buprenorphine | 25.0 |
| Pentazocine | 3-6 |
| Nalbuphine | 0.1 |

TABLE 1   (continued)

|  | P |
|---|---|
| Butorphanol | 0.5 |

Although the device of the invention may contain from 11 to 3000 mg of morphine or equivalent quantity of another opiate/opioid analgesic, the preferred dose is from 15 to 3000 mg morphine or from $\frac{15}{P}$ to $\frac{3000}{P}$ mg of said other analgesic. More preferred is from 15 to 300 mg morphine or from $\frac{15}{P}$ to $\frac{3000}{P}$ of said other analgesic and the most preferred dose is 100 mg morphine or $\frac{100}{P}$ mg of said other analgesic.
Ideally the device should deliver to the body between about 0.005 to about 0.1 mg/kg/per hour for a period of 24 to 144 hours and the concentration of opiate/opioid may be appropriately adjusted so that this is so. The doses of opiate/opioid analgesic specified as appropriate above assume that the patient is "opiate naive" i.e is not opiate addicted. In addicted patients the device should deliver from 0.1 to 1.0 mg/kg/hour.

Suitable opiate/opioid analgesics are listed above and include the salts of those compounds. Preferred for use in the transdermal device of the invention is morphine and in particular the hydrochloride, sulphate, ascorbate, acetate or tartrate salts thereof. In the case of a device comprising hydromorphone, the hydrochloride salt is preferred.

The opiate/opioid analgesic may be incorporated within the device in an aqueous solution or another solvent may be used. The solution may be saturated, unsaturated or supersaturated. It may also be present in other physical forms, such as, for example, dispersed in a polymer matrix. It is also envisaged that the device may contain the opiate/opioid analgesic in a dried, preferably freeze-dried, form which is reconstituted in a suitable solvent immediately prior to drug delivery. Such a device has particular advantages from the point of view of long-term storage and sterility.

Preferably, the opiate/opioid analgesic is mixed with from 1 to 10 mg of sodium pyrosulphite.

The device may be one which, in addition to inclusion of the opiate/opioid analgesic has means for creating the de-epithelialized lesion by forming a suction blister. Suitable devices are described in the Applicant's published International Patent Application Nos WO 92/11879 and WO 95/15783. Alternatively, the device may be one incorporating the analgesic to be applied to a de-epithelialized lesion which has been generated independently. In particular the device may be a patch or flexible plaster of laminar structure in which the analgesic is incorporated, for example, in a polymer matrix. Suitable devices are described in the Applicant's published International Patent Application No WO 95/30410. A further alternative is the use of a device which is capable of forming the de-epithelialized lesion and subsequently apply a drug-loaded plaster or patch to the lesion. Such a device is described in the Applicant's International Application No WO 95/15783.

The invention is also directed to a method of administration of an opiate/opioid analgesic to the human or animal body which comprises forming a de-epithelialized shin lesion of a standard predetermined size and applying to the lesion a device as described above. As an alternative a single device can be applied to intact skin which both forms the de-epithelialized lesion and subsequently delivers to that lesion, morphine or another opiate/opioid analgesic at the rate described above.

The method of the invention is suitable for delivery of opiates/opioids to neonates and small children.

The invention will now be described by way of example with reference to the drawings and examples in which:

Figure 1 shows in cross-section, a schematic view of a device in accordance with the invention,'
Figure 2 shows the device of Figure 1 in a first position following removal of the roof of the suction blister,
Figure 3 shows the device of Figure 1 in a second position to deliver the requisite dose of opiate/opioid analgesic to the skin lesion;
Figure 4 shows the device of Figure 3 in a third position in which the reservoir containing said opiate/opioid analgesic is closed to the skin lesion;
Figure 5 shows a connectable vacuum source and indicator means for use in conjunction with the device in accordance with the invention;
Figure 6 shows a modification of the device of Figure 1 in which the drug reservoir is replaced by a patch containing said opiate/opioid analgesic;
Figure 6a is an underneath plan view of the device of Figure 6;
Figure 7 shows the device of Figure 6 in a first position following removal of the roof of the suction blister;
Figure 8 shows the device of Figure 6 in a second position with the protective film removed from the adhesive layer of the patch;
Figure 9 shows the device of Figure 6 in a third position with the patch in alignment with the de-epithelialized skin lesion;
Figure 10 shows the device of Figure 6 in a third position with the actuator depressed to apply the patch to the de-epithelialized skin lesion;

Figure 11 shows the patch *in situ* on the de-epithelialized skin lesion;
Figure 12 shows an embodiment of the invention in which the opiate/opioid is incorporated in dried form;
Figure 13 shows another embodiment of the invention in which the opiate/opioid is incorporated in dried form;
Figure 14 shows a device similar to that shown in Figure 12 with apparatus for applying a reconstituting solution to the dried opiate/opioid;
Figure 15 is a schematic representation of the vasculature and nerves of the dermis showing splitting of the epidermis under suction;

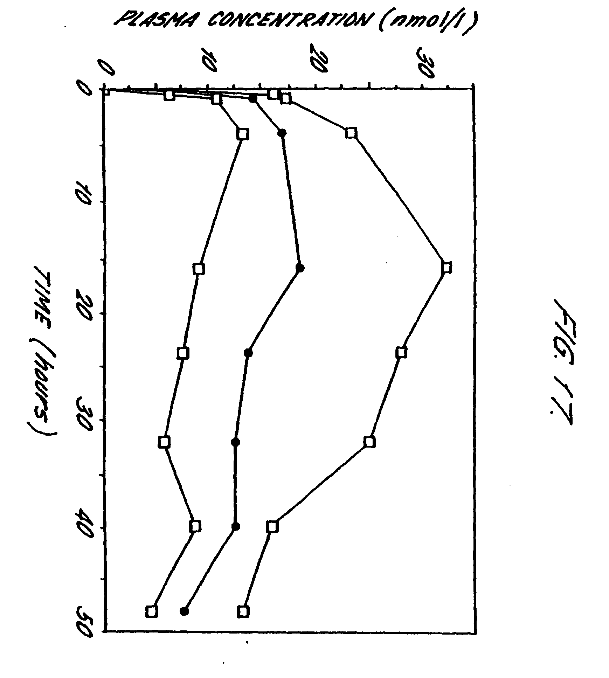

Figure 16 shows plasma levels of morphine and its metabolite M6G over 48 hours following use of a device in accordance with the invention on women having elective hysterectomy and Figure 17 shows plasma levels of morphine following use of a device in accordance with the invention on patients having elective cardiac surgery. □—□ represents the lowest and highest concentration at each time point and •—• the mean value.

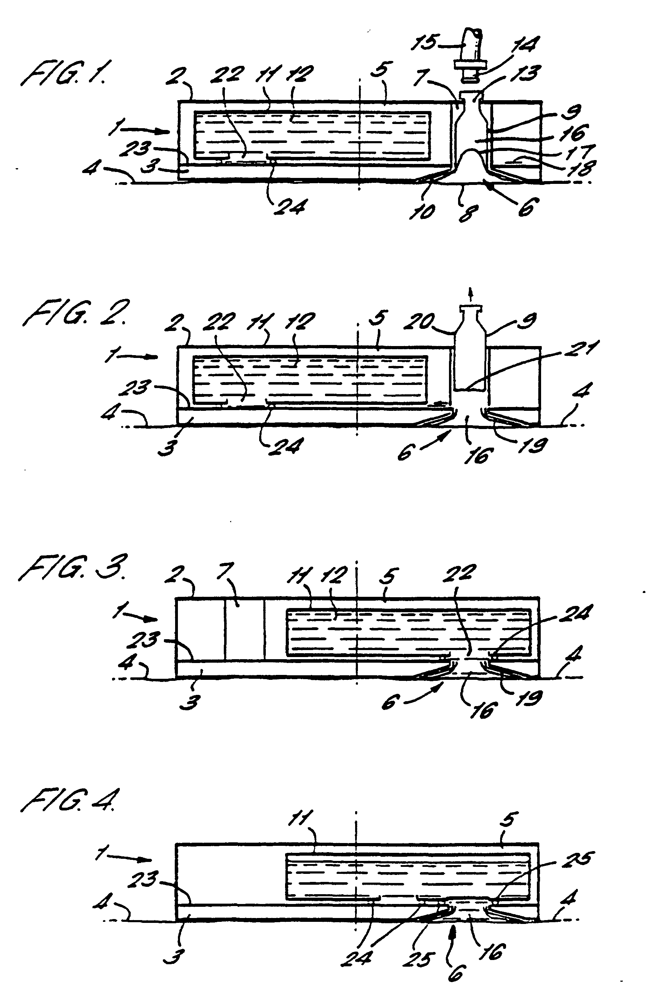

Figure 1 shows schematically a device in accordance with the invention which is suitable for transdermal delivery of an opiate/opioid analgesic over a period of 48 to 144 hours but which also includes means for forming the de-epithelialized lesion. In particular it permits the application of suction to a particular area of skin to form a suction blister and facilitates disruption of the blister to leave the dermis exposed.

The device 1 comprises a housing 2 consisting of a base 3 secured in contact with a patient's skin 4 and having a rotatable portion 5. The base 3 is disc shaped and the rotatable portion 5 generally cylindrical and coupled to the base so as to be rotatable relative to the base about its cylindrical axis in continuous sliding contact with the base.

A circular aperture 6 is defined in the base 3 at a location eccentric relative to the cylindrical axis of the rotatable portion 5. In the rest position of the device shown in Figure 1, a cylindrical access port 7 defined in the rotatable portion 5 is aligned in communication with the aperture 6 such that a circular area of skin 8 is accessible through the device 1.

A suction cup 9 is located in the access port 7 and has a flared lip 10 of greater diameter than the port 7 such that the suction cup is captively retained. The base 3 is recessed peripherally of the aperture 6 to accommodate the lip 10. The internal surfaces of the cup 9 are coated with acrylic adhesive so that, once a suction blister is formed in the cup, the surface of the blister will adhere to the cup, thereby maintaining the blister in an elevated position. This will tend to prevent collapse of the blister in the event of accidental rupture.

The rotatable portion 5 also accommodates a reservoir 11 containing the opiate/opioid analgesic in solution in an amount as specified above in accordance with the invention, the reservoir 11 being isolated from the access port 7 in the position shown in Figure 1.

The suction cup 9 has a female connector 13 which is engagable with a male connector 14 of a suction tube 15 via which suction can be applied in use to a suction chamber 16 defined within the suction cup 9. In Figure 1, the device is shown in its rest position following the application of suction within the chamber 16 for a period sufficient to result in the formation of a suction blister 17, following which the male and female connectors 13 and 14 have been disconnected from one another to admit air at ambient pressure to the chamber 16.

The device 1 also includes a blade 18 which extends radially with respect to the cylindrical axis of the rotatable portion 5 and which is movable by rotation about the cylindrical axis in a plane defined by the interface between the base 3 and the rotatable portion 5.

In Figure 2 the operation of the blade 18 is illustrated in that it has been moved arcuately so as to cut through the suction cup 9 at a location which is intermediate the roof of the suction blister 17 and the aperture 6. By this cutting action, the suction cup 9 is severed into a captive portion 19 extending through the base 3 and a removable portion 20 extending through the rotatable portion 5 and to which the roof 21 of suction blister 17 remains adhered.

By withdrawing the removable portion 20 from the access port 7, the roof 21 may thereby be disposed of. Since the removable portion 20 is severed at the interface between the base 3 and the rotatable portion 5, the severing action of the blade 18 thereby allows the rotatable portion 5 to be subsequently movable by rotation relative to the base 3 whereas previously the presence of the suction cup extending through the aperture 6 and access port 7 prevented such relative rotational movement about the cylindrical axis.

The reservoir 11 has an outlet port 22 which in the rest position of the rotatable portion 5 as shown in Figures 1 and 2 is closed by an upper surface 23 of the base 3, a continuous O-ring seal 24 being interposed between the surface 23 and the rotatable portion 5 to prevent peripheral leakage from the outlet port 22. Following the severing of the suction cup 9, the rotatable portion 5 is rotated into a second position shown in Figure 3 in which the outlet port 22 is brought into registration with the aperture 6, the location of the outlet port 22 being spaced radially from the cylindrical axis of the rotatable portion 5 about which it is rotated.

The O-ring seal 24 is maintained in a fixed position relative to the rotatable portion 5 so that in this second position it forms a peripheral barrier between the rotatable portion 5 and the upper surface 23 of the base 3. The opiate/opioid analgesic, for example morphine, within the reservoir 11 then enters the chamber 16 and comes into contact with the area of skin 8 which has been de-epithelialized following removal of the suction blister 17. The device 1 is retained in this second position during a drug delivery phase of operation which may be for a period of up to 144 hours during which the analgesic is absorbed into the patient.

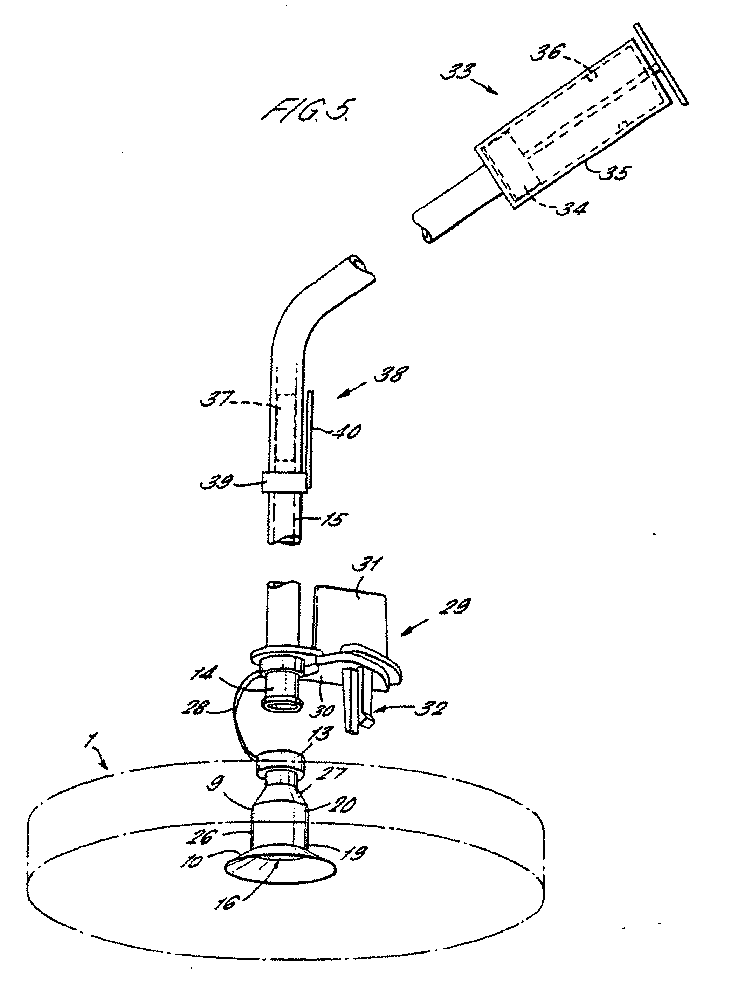

On completion of this phase, the rotatable portion 5 is again rotated and moved into a third position shown in Figure 4. In this third position, the outlet port 22 of the reservoir 11 is again closed by the upper surface 23, assisted by the sealing action of the O-ring seal 24. A second O-ring seal 25 is brought into peripheral sealing engagement between the upper surface 23 and the rotatable portion 5 at a location peripheral to the aperture 6 thereby providing an air tight seal to the chamber 16.

The rotatable portion 5 may subsequently be returned to the second position should further drug delivery be required or the device 1 may be removed from the patient on completion of the procedure.

In Figure 5 the suction cup 9 is shown to comprise a cylindrical portion 26 defining the chamber 16 and which in use is severed radially by action of the blade 18 into captive and removable portions 19 and 20. The internal surfaces of the suction cup 9 are coated with an acrylic adhesive incorporating random oriented polyester fibres.

The removable portion 20 terminates in the female connector 13 to which it is connected by a frustoconical tapered portion 27. The male connector 14 is connected by a flexible web 28 to the female connector 13 such that when disconnected from one another they are retained in loose association. An arming device 29 is connected to the male connector 14 and consists of a plate 30 through which the suction tube 15 passes, the plate being formed integrally with an outwardly projecting handle 31 and an oppositely projecting bifurcated arming pin 32. The arming device 29 is shaped such that it must be inserted within the rotatable portion 5 in order for the male and female connectors 14 and 13 to be engaged, the presence of the inserted arming pin 32 being arranged to prevent movement of the blade 18 from its initial position as shown in Figure 1. This arrangement thereby ensures that the blade 18 cannot be moved until the arming device 29 has been disengaged from the rotatable portion 5 and this disengagement also necessitates disengagement of the male and female connectors 14 and 13 so that suction can no longer be maintained in the chamber 16. This is a safety feature of the device 1 which is intended to avoid cutting the suction blister 17 while there is any suction within the chamber 16 which could displace the underlying dermis into a position in which it extends into the chamber sufficiently to be damaged by the blade.

The suction tube 15 is connected to a syringe 33 comprising a piston 34 slidable within a cylinder 35 and spring biassed into the position shown in Figure 5 in which the syringe volume is a minimum. The syringe is provided with a locking mechanism 36 enabling the piston 34 to be held in a withdrawn position corresponding to maximum syringe volume so that suction can be applied to the chamber 16 by engaging the male and female connectors 14 and 13 and then withdrawing the piston and locking the piston in place by means of the locking mechanism.

The suction tube 15 is formed of a transparent and flexible plastics material and contains a slug of liquid 37 forming part of an indicator 38.

The indicator 38 comprises a clamping ring 39 which is a tight fit on the external surface of the suction tube 15 but can be adjusted in position along the length of the tube so as to bring into registration with the slug of liquid 37 a linear scale 40. When suction is initially applied to the chamber 16 by action of the syringe 33, the position of the slug of liquid 37 will move due to displacement of air along the tube 15 to a new position and the operator using the device 1 at this time adjusts the position of the clamping ring 39 such that the end of the slug of liquid 37 is aligned with a zero marking on the scale 40. Suction is maintained with the chamber 16 during a blister forming period in which the suction blister 17 will progressively form and grow in size until it extends into the cylindrical portion 26. In doing so the blister 17 will displace air within the tube 15 and consequently the slug of liquid 37 will be linearly displaced relative to the scale 40. The scale 40 is calibrated such that the operator is able to determine the extent of displacement of the slug of liquid 37 corresponding to the blister 17 being fully formed to a predetermined level at which a predetermined volumetric displacement within the chamber 16 is achieved.

By visual inspection of the indicator 38 it is therefore possible for the operator to determine when the blister forming phase of operation is completed.

At this stage the operator will grip the handle 31 and pull the arming device 29 so as to withdraw the arming pin 32 and at the same time to disconnect the male connector 14 from the female connecter 17. Suction within the chamber 16 will then be lost.

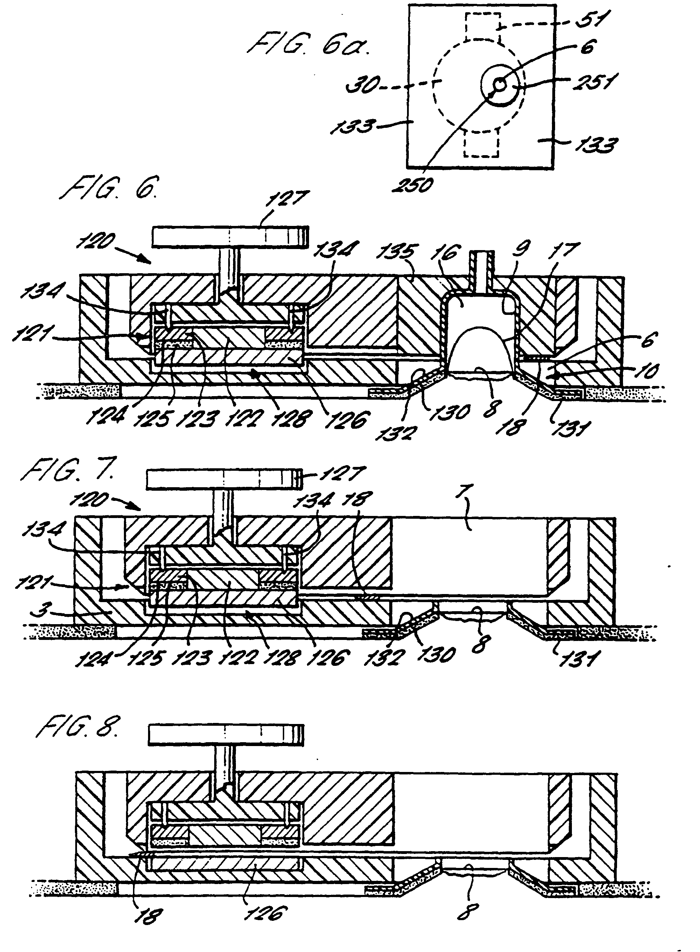

As an alternative to a device of the type described in Figures 1 to 4 the opiate/opioid analgesic may be delivered via a transdermal patch or plaster of laminar structure. The composition of such patches is discussed in more detail below. They may be applied manually, directly to a de-epithelialized lesion previously prepared or they may be applied automatically by a device which is also capable for forming the de-epithelialized lesion. Such a device is shown in Figures 6 to 11.

The device 1 is modified in Figure 6 to include a patch applicator 120 which is operable to apply a patch 121 to the area of skin 8 following de-epithelialization while the device 1 remains *in situ.*

The patch 121 consists of a disc shaped central element 122 which is to be reactively engagable with the de-epithelialized area of skin 8 and which contains the appropriate dose of opiate/opioid analgesic in a manner described further below. Attached peripherally to the central element 122 is a relatively rigid support ring 123 having on its underside 124 an adhesive layer 125.

A protective film 126 overlays the underside 124 of the support ring 123 and the central element 122 thereby maintaining the efficacy of the adhesive layer 125 prior to use and sealing the central element 122.

The support ring 123 is held in position by means of pins 134 which are mounted on the rotatable portion 5 and which pierce the support ring 123. An actuator 127 is arranged so as to contact the support ring with the pins 134 extending slidably through the actuator such that by movement of the actuator the support ring can be disengaged from the pins. The patch 121 is received in a patch chamber 128 defined by the rotatable portion 5 which in the initial position of the rotatable portion communicates with a co-operating recess 129 formed in the upper surface of the base 3.

The actuator 127 projects upwardly and clear of the rotatable portion 5 so as to be externally accessible to the user and is spring loaded into a raised position in which the patch 121 is suspended clear of the base 3.

In this initial position, the interface between the protective film 126 and the adhesive layer 125 is in alignment with the locus of movement of the blade 18 between the base 3 and rotatable portion 5.

The actuator 127 is reciprocatable in a direction towards and away from the base 3 so as to be operable to displace support ring 123 and with it the patch 121 in a direction at right angles to the plane of the base 3. The device of Figure 6 has a base 3 which defines an aperture 6 which is of enlarged diameter sufficient to accommodate passage of the patch 121 and receives a suction cup 9 having a lip 10 whose underside is coated with adhesive layer 130. An annular plug 135 of resilient foam material is inserted between the suction cup 9 and the walls of the access port 7 so as to retain the suction cup in coaxial relationship with the access port and retain the suction cup relative to the base 3 and rotatable portion 5 prior to use. The lip 10 comprises an outer annular region 131 extending radially and at right angles to the cylindrical axis of the suction chamber 16 and further comprises an inner annular region 132 which is frustoconical in shape. It should be noted that the degree of conicity of the inner annular region in Figure 6 is exaggerated for clarity and that the axial extent to which the inner annular region projects is typically a fraction of 1 millimetre.

As shown in the underneath plan view of Figure 6a, the base 3 is attachable to the skin 4 of a user by means of an adhesive tape 133. A circular opening 250 is provided in the tape 133 to coincide with the aperture 6 within which the skin site is to be accessed. An annular region 251 surrounding the opening 250 is provided with an acrylic adhesive coating so as to be impervious to body fluids and the remaining area of the tape receives a hydrocoloidal adhesive coating.

The suction cup 9 and the base 3 are thereby independently securable to the skin 4 by means of the adhesive layer 130 and the adhesive tape 133 respectively.

In use, the suction cup 9 is used to form a suction blister 17 as described above with reference to the device of Figure 1. During the application of the device to the skin, the skin area 8 will adhere to the adhesive layer 130 and during the blister forming period the skin area 8 will tend to remain raised within the inner annular region 132 of the lip 10 by suction.

The device is then actuated by rotation of the actuating ring 46 as shown in Figure 1 to move blade 18 through the suction cup 9 thereby severing both the cup and the blister 17 and exposing a de-epithelialized area of skin 8 within the aperture 6.

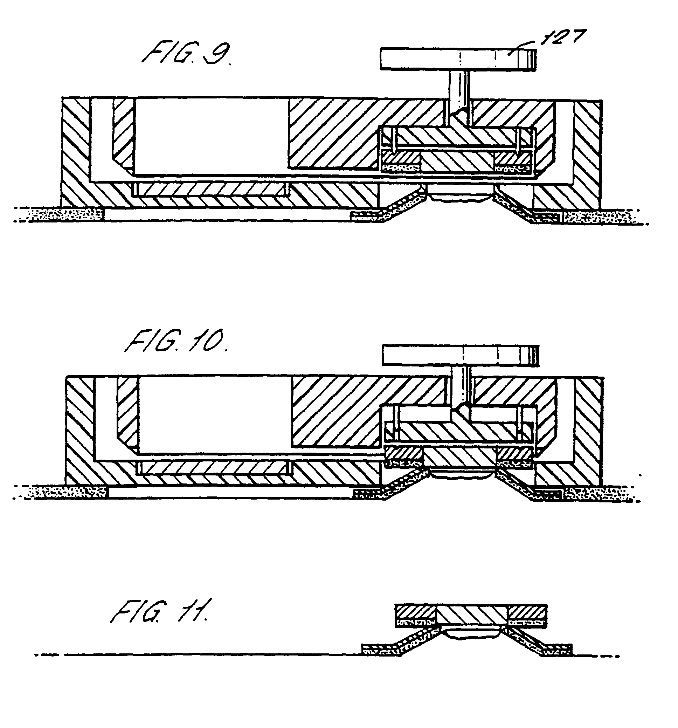

The actuating ring 46 is further rotated to drive the blade 18 through the patch chamber 128 so as to separate the protective film 126 from the adhesive layer 125 as shown in Figure 8. A thickened blade 18 of wedge shaped cross section may therefore be advantageously used in this embodiment in order to facilitate separation. The discarded protective film 126 is then allowed to fall into the recess 129 where it remains. The actuating ring 46 is further rotated to engage and rotate the rotatable portion 5 of the device and to move the patch chamber 128 into registration with the aperture 6 as shown in Figure 9.

The actuator 127 is then depressed so as to displace the patch 121 axially within the patch chamber 128 towards and into contact with the area of skin 8, the diameter of the central element 122 being dimensioned so as to be slightly greater then the de-epithelialized area 8 of skin exposed when the blister is disrupted.

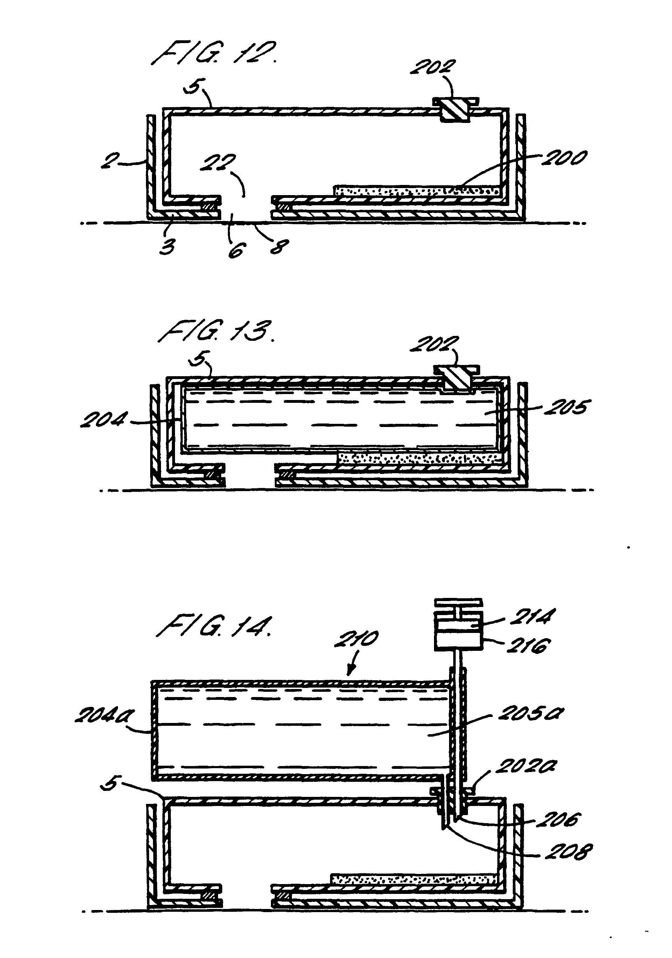

In depressing the actuator 127 as shown in Figure 10, the support ring 123 is dissociated from the attachment pins 134 which remain stationary relative to the rotatable portion 5. The actuator 127 when subsequently retracted no longer carries with it the support ring 123 and the patch 121 remains *in situ* in contact with the area of skin 8. The adhesive tape 133 is then dissociated from the skin 4 and the base 3 dissociated from the skin so that the device can be lifted clear. The patch 121 remains *in situ* as shown in Figure 11 with the central element 122 remaining in intimate contact with the de-epithelialized area of skin 8.

The device 1 of Figures 6 to 11 enables a newly formed de-epithelialized site to be covered prior to removal of the device 1 thereby avoiding exposure of the dermis to atmosphere. This techniques also ensures that the patch 121 is automatically positioned accurately in alignment with the de-epithelialized lesion.

The patch 121 is a self contained means for administering the appropriate dose of opiate/opioid analgesic.

A patch or plaster which forms the transdermal device of the invention, whether or not of suitable dimensions for application by a device as shown in Figures 6 to 11, may have a variety of different structures which are well-known in the art. A typical patch is a flexible device of laminar structure having a backing layer of an impermeable material such as, for example an aluminized polyester film, laminated to a reservoir for the active agent, in this case an opiate/opioid analgesic. The reservoir may comprise a chamber containing said opiate or opioid analgesic in a solution. The solution may be unsaturated, saturated or supersaturated depending on the solvent concerned and so long as the total amount of drug is always 11 mg or greater in the case of morphine and $\frac{11}{P}$ mg or greater in the case of another opiate/opioid analgesic.

As an alternative to the chamber as described above the impermeable backing layer of the patch may be laminated to a material forming an inert porous matrix in which said opiate/opioid analgesic is impregnated or otherwise dispersed in an appropriate amount. Many materials are known to be suitable for the preparation of such a matrix. For example, hydrogels which comprise a very large class of materials with characteristic swelling and diffusional properties can be used. Biocompatible hydrogels for drug delivery applications may be based on polymers like, hydroxyethylmethacrylate, polyethylene glycols and polyethylene oxide. These materials can be crosslinked by radiation or by other known methods to provide water-insolubility. In manufacture a watersoluble opiate/opioid analgesic such as morphine may be mixed with the dry hydrogel which absorbs water into the matrix and so swells.

Charged polymers of the type used in ion-exchange and electrodialysis may also provide a suitable porous matrix for the analgesic. Positive ionic groups e.g. tertiary ammonium groups or negative ionic groups e.g. sulphonic acid groups, are coupled to the polymer backbone.

Hydrocolloids manufactured by direct compression of powder such as methylhydroxypropylcellulose or polyvinyl alcohol can be useful as an inert porous matrix for the active agent as well as numerous other synthetic and natural polymers known to the skilled man.

The porous matrix containing the appropriate quantity of analgesic in accordance with the invention must be adhered to the skin so as to be maintained in contact with the de-epithelialized lesion. An adhesive layer may therefore be laminated to the porous matrix. As an alternative however it is particularly convenient if the polymer used to form the porous matrix is in fact an adhesive material. Therefore, particularly preferred for transdermal use are acrylate, silicone and polyisobutylene adhesives which are capable of forming a porous matrix suitable as a reservoir for the analgesic. Hydrocolloid adhesives are also useful because they can absorb water from the intact skin layer without reducing the integrity of the adhesive. This is particularly important for the patches of the invention since prolonged administration of up to 148 hours at a steady rate is a particular advantage.

One further standard feature of a transdermal patch or plaster is a siliconised release liner which is adhered to the adhesive layer and then stripped off prior to application of the patch to the skin.

In accordance with one particular embodiment of the device of the present invention, whether it be of the general type described in WO92/11879, WO95/15783 or Figures 1 to 4 herein or a flexible plaster or patch such as that described in WO95/15783, the opiate/opioid analgesic is incorporated in a dried, preferably freeze dried, form. A suitable solvent for the opiate/opioid is then introduced into the device to produce a solution of appropriate concentration at the time of use. It will be appreciated that where the opiate/opioid is in dried form the shelf-life of the device is longer and it is less susceptible to bacterial contamination.

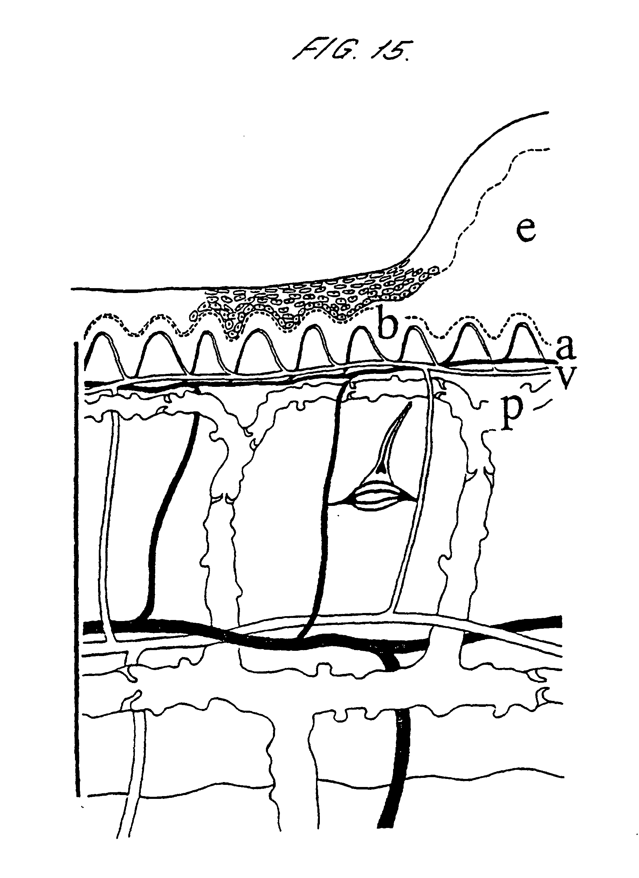

Suitable devices in accordance with this embodiment of the invention are shown in Figures 12 to 14.

The device of Figure 12 comprises a housing 2 consisting of a base 3 which can be secured in contact with the patient's skin and having for example a rotatable portion 5. The rotatable portion 5 incorporates the freeze-dried opiate/opioid 200 and is provided with a removable plug 202. As shown in Figure 12 the rotatable portion includes an aperture 22 capable of being aligned with the aperture 6 in the base 3, which aperture is placed over a de-epitheliclized skin lesion 8.

In use, after applying the base of the housing to the skin, the plug 202 is removed and a liquid for reconstitution of the freeze-dried material 200 is introduced. Thereafter, the device delivers the opiate/opioid analgesic to the patient through the de-dpithelidized lesion in the same manner as the other embodiments of the device described herein.

An alternative embodiment incorporating the opiate/opioid in freeze-dried form is shown in Figure 13. In this case the liquid 205 for reconstitution of the freeze-dried material is present within the device in a reservoir 204 housed entirely within the rotatable portion 5. The reservoir 204 is constructed of glass or other breakable or rupturable material so that by applying pressure to the plug 202 the compartment breaks or ruptures and the reconstitution liquid comes into contact with the freeze-dried material 200.

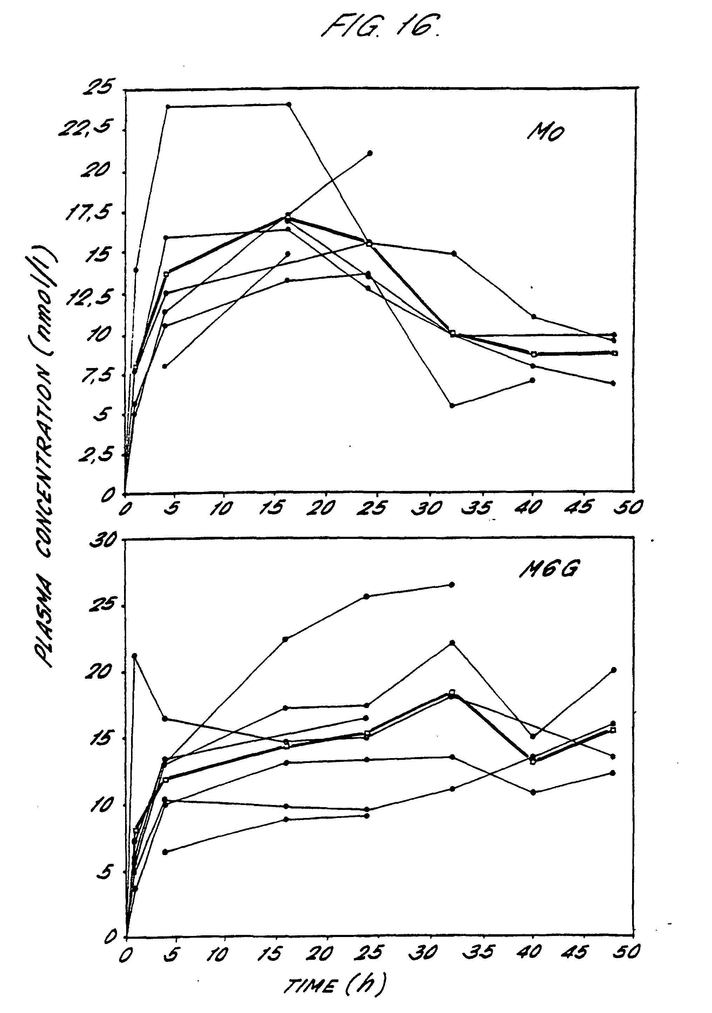

Yet a further embodiment is shown in Figure 14. Here an elastic plug 202a is provided in the rotatable portion 5 which is capable of receiving cannulas 206 and 208. Said cannulas form part of an apparatus 210 for supplying tne reconstitution liquid to the freeze-dried material in the rotatable portion 5. The apparatus 210 comprises a reservoir 204a which holds the reconstitution liquid 205a, the reservoir having an outlet 212 to cannula 208, the housing for the reservoir 204a being integral therewith a tube or cannula 206. The cannulas 208 and 206 can be pushed through the elastic plug 202a so as to have their open ends deposed within the rotatable portion 5. At the end opposite to that which is pushed through the plug, the cannula 206 is provided with a piston 214 movable within a housing 216.

Figure 14 shows the apparatus 210 in use with a transdermal device in accordance with this embodiment of the invention. After the transdermal device comprising the freeze-dried opiate/opioid analgesic is applied to the patient's skin the piston 214 is moved upwards within the housing 216. This creates a partial vacuum within the rotatable portion 5 so that the solution in the reservoir 204a is sucked therein so reconstituting the freeze-dried material and bringing it into direct contact with the de-epithehalized lesion 8.

It will be appreciated that if the arrangement of the transdermal device is such that when the device is applied to the skin the reservoir containing the opiate/opioid solution is not open to the de-epithehalized lesion, for any of the devices described above, the freeze-dried material could be reconstituted with an appropriate solvent before the device is actually applied.

In a further alternative to the embodiment described above the dried material may comprise not only the opiate/opioid analgesic but also another dried material which is a carrier or matrix for the analgesic. On addition of a suitable liquid both the analgesic and the carrier are reconstituted.

The carrier may for example be a polymer such as those described hereinbefore. The opioid/opiate may be directly bonded to the said carrier or in salt form or the said carrier may contain suitable acids for forming opioid/opiate salts once a solvent, preferably water, is added. The matrix may itself be dissolved in solution or it may be partially or completely insoluble.

Where another material is mixed with the dried opiate/opioid analgesic in this manner, depending on the nature of the reconstituted carrier, the rate of the release of the analgesic to the de-epithelialized lesion can be controlled, in accordance with state of the art slow release technology.

The diffusion properties of opioid/opiate from a slow release preparation within the matrix or carrier may be selected in such a way that the rate of entry of morphine or salt thereof into the solution is such that it matches the rate of entry of drug into the body. This ensures unchanged rate of drug delivery over time.

Lactate, saccharides, hydrogel polymers, carbomer particles and cyclodextrine tubes may be used for forming slow release preparations with opioid/opiate analgesics.

Example 1

TRANSDERMAL ADMINISTRATION OF MORPHINE HYDROCHLORIDE

This experiment was undertaken in consenting volunteers after approval by the Ethics Committee of Lund University and by the Swedish Medical Products Agency. The morphine study included seven women aged 40-61 years (mean: 49 years) scheduled to undergo elective hysterectomy. They were otherwise healthy.

The disposable "cellpatch" used clinically (Epiport Pain Relief AB, Malmo, Sweden), shown in Figures 1 to 4, allowed formation of a 6mm mini-erosion in the skin and continuous drug delivery. The 5.5 ml cell was filled with drug and plugged. After the skin was lightly washed with chlorhexidine solution (0.5 mg/ml) and dried the device was applied. It was operated as follows: The volume expander piston was withdrawn and locked, creating a relative vacuum in the suction cup of 200mm Hg below atmospheric. This vacuum exposure split the epidermis at a level deep to the skin barrier but superficial to the microvasculature and nerves of the dermis, see Fig. 12. This caused no discomfort to the patient. The split became filled with plasma filtrate and a vesicle formed which increased in size until it filled the suction cup. The volume displacement pushed the indicator slug away from the vesicle. When the slug reached the mark on the tube wall the vesicle formation was complete. Lock, tube and volume expander were then removed from the suction cup as a single unit, letting in air. Drug delivery was initiated by rotating the rotatable portion or operating ring clockwise about one complete turn to its second position through the first position where the blade was drawn through the suction cup at the level of its aperture towards the skin. Both the cup and the contained vesicle were circumferentially ablated, forming the erosion. As the rotation was continued to the second position the drug cell engaged the ring and slid within the frame until its bore resided over the erosion, exposing it to the drug solution. The delivery was stopped by anticlockwise rotation of the ring to complete stop. The Cellpatch was then removed.

After premedication with pethidine hydrochloride ("Petidin", ACO, Sweden) general anaesthesia was induced with thiopental followed by orotracheal intubation and mechanical ventilation with $N_2O/O_2$. Pethidine hydrochloride and fentanyl citrate ("Leptanal", Janssen, Belgium) was used as required during operation, and pethidine hydrochloride postoperatively. In some patients non-opioid analgesics were used additionally. There were no surgical complications.

The Cellpatch was filled with 100mg morphine preoperatively (26.6μ, 5.0ml morphine hydrochloride in aqueous solution (20 mg/ml, Apoteksbolaget, Sweden) and applied preoperatively on the volar aspect of the exposed forearm at induction of anaesthesia. Additionally, a light, circumferential bandage was used. Morphine delivery, started as soon as the patient entered the postoperative unit, was planned to last for 48 hours. Postoperative monitoring of blood pressure, heart rate, respiratory rate and oxygen saturation followed the routine of the unit. Pulse-oximetry was used during the initial 24 hours and during the second night which the patient also spent in the unit. The respiratory alarm was set at 10/min, pulse rate at 55/min and oxygenation alarm at 93% $O_2$ saturation.

At 24h the women were questioned about any inconvenience or pain caused by the Cellpatch or the indwelling i.v. cannula. The patch was moved with the underlying skins as a provocation test. At 48h they were asked to rate their preference on a 5 point scale for receiving drug either by Cellpatch or cannula. The delivery site was inspected directly upon removal of the patch, 6 - 14 days later and at follow up 2 - 6 months later. Samples of venous blood were taken by repeated cannulation in arm veins directly before starting transdermal morphine at 0 min and at 1h, 4h, 16h, 24h, 32h, 40h and 48h. The drug cell was sampled at 48h for bacteriological testing.

Venous blood samples were collected from arm veins in heparinized "Vacutainer" tubes which were centrifuged within 30min. The plasma was then separated and frozen. Morphine and M6G, a metabolite of morphine, were analysed using an HPLC method with electrochemical and ultraviolet detection. The morphine solution remaining in the drug cell after therapy was cultured on an aerobically incubated blood agar plate, on an anaerobically incubated blood agar plate and on a haematin agar plate. All plates were incubated at 37°C for at least 48h. Another sample of the solution was put into a culture tube with tryptic soy broth (Oxoid, UK). The broth was incubated for 24h and then subcultured on a haematin agar plate incubated aerobically at 37°C for 48h.

Four of the seven patients were followed for 48h. The three that dropped out did so at 24h (n=2) or 32h, complaining of pain and discomfort in connection with the blood sampling and/or not wanting to spend a second night being monitored in the postoperative unit.

Results

The Cellpatch was considered easy to use. The patches adhered completely to the skin for the duration of therapy. The patients complied readily. While the Cellpatch caused no discomfort the i.v. cannula produced intermittent light pain and some sense of stiffness which could be further provoked when the women moved the arm or when the projecting part of the cannula was inadvertently moved. According to their ratings (4-5 on the 5-point scale), all women preferred to receive drugs through the Cellpatch rather than through a cannula. When the patch was removed there was no sign of inflammation in the erosion or the adjacent skin, and the skin under the patch was normal. A week after removal of the cellpatch the epidermis had regenerated, and the remaining erythema was considered trivial by all the women. At follow-up, close examination revealed a tiny, fading pigmentation which had become almost invisible in the 3 patients treated initially.

Of the seven samples taken for bacteriological testing, six were negative in all cultures. In one sample low counts (90 CFU/ml and 120 CFU/ml, respectively) of two different strains of coagulase negative staphylococci (CNS) were found on the aerobically incubated plates.

The plasma concentrations of morphine and M6G for all the patients are shown in Fig. 13. Some blood samples were inadvertently taken from a vein ipsilateral to the Cellpatch. The clearly artificial morphine values from these samples were omitted, while M6G values were recorded. Morphine delivery was associated with a mean $C_{max}$ value of 17.3 ± 3.7 nmol/l, with a mean $t_{max}$ value of 16.7h (range 4h to 24h). The mean morphine concentration was 8.0 ± 3.5 nmol/l (SD) at 1h. At 32 and 48h the values were 9.8 ± 3.8 nmol/l and 8.5 ± 1.7 nmol/l, respectively. The mean $C_{max}$ value of M6G was 17.6 ± 5.6 nmol/l and the $t_{max}$ value ranged from 1 to 48h (mean 25.5h). During the initial 24h the patients received 166 mg pethidine hydrochloride (mean)(absolute range: 50 mg - 295 mg). During the following 24h, 37 mg pethidin was given (50 mg - 100 mg). The limits set for postoperative monitoring were not exceeded, and the oxygen saturation remained normal during the test period.

Example 2

TRANSDERMAL ADMINISTRATION OF MORPHINE HYDROCHLORIDE

Passive transdermal administration of morphine through de-epithelialized skin was given, in the dose and manner described in Example 1, to 8 patients aged from 42 to 68 years following elective trans-thoracic coronary surgery. The patients differed markedly in weight from 56 to 110 kg. The morphine delivery was started in the morning following the operation and continued for a period of 48 hours. Blood levels of morphine were determined as described in Example 1 over the 48 hour period. The results are shown in Figure 14. After 48 hours 32 ± 6 mg of the initial 100 mg morphine dose in the drug cell was found to be absorbed.

The clinical pilot trial of the above examples 1 and 2 demonstrate the feasibility of administering morphine transdermally through de-epithelialized skin for postoperative pain relief. The device of the invention performed well in the demanding conditions of the postoperative unit, and compliance was excellent. The sustained plasma levels of morphine and the low inter-individual variation corroborate closely the findings in a previous pharmacokinetic study and are comparable with those obtained with a protracted infusion. The technique would be exceptionally safe: Systemic absorption, restricted by the small size of the mini - erosion and occurring by diffusion through an intact vascular membrane which includes the lymphatics, is rate-limited and reproducible. Overdosing by inadvertent acute dispersal of the contents of the cell into the body cannot occur by this mechanism. There is no foreign body penetration into the tissues which may channel bacteria. The technique is therefore suitable for use with opiate/opioid analgesics which currently need to be given by injection or infusion. By eliminating the epidermal barrier, controllable absorption of a drug through a limited area of dermal microvascular wall can be achieved by diffusion using an aqueous solution applied topically. Assuming unchanged microvascular flow and volume of opiate/opioid within the dermis at the delivery site, the absolute rate of absorption relates to the concentration of drug in the interstitium and is limited by the maximal aqueous solubility of the drug. The total dose in the cell decides the duration one may proceed efficiently with a certain rate of supply.

Morphine is not normally absorbed transdermally. The size of a conventional morphine patch on intact skin would need to be 62500 cm$^2$ to provide the equivalent of an i.m. injection of 10 mg morphine. Bioavailability achieved by other non-invasive routes ranges from 20 to 40 per cent, and inter-individual variation is wide. In the previous pharmacokinetic study carried out by the applicant, the drug cell contained to mg morphine (20mg/ml), delivery was continued for 24h, and the absolute bioavailability ranged between 65 and 85 per cent (mean 75 per cent). The $C_{max}$ levels were similar in both studies, but higher concentrations were maintained much longer in patients as a consequence of increasing the total amount of the drug. This was not to be expected from the previous study. The plasma concentrations and the clinical findings demonstrate the feasibility of using a morphine containing transdermal device for providing basal postoperative pain relief over a prolonged period. It is obvious that more potent opioid therapy, capable of delivery on an on-off basis, and thus adjustable to the patient's total need for pain relief, is enabled by this method.

The erosion itself heals in about a week independent of its size, since epithelial migration occurs not only from its edge but from the scattered remnants of the adnexal structures. The benign course taken corresponds to that of a natural or traumatically induced skin vesicle. The absence of any significant bacterial growth corroborates the accumulated experience gleaned from current clinical studies and from pharmacokinetic studies on opiates in volunteers. Clinical use of the mini-erosion also derives support from hundreds of studies where suctioning has been used in humans as a means of splitting off the epidermis, mainly for the purposes of dermatological research and for assessment of drug concentrations in the peripheral compartment. In these studies a plurality of actual blisters, covering areas of skin much larger than that of the small vesicle that we propose using, were utilised. No complications were reported. Cosmetically, the skin was normalised.

Example 3

TRANSDERMAL ADMINISTRATION OF HYDROMORPHONE

30 mg of hydromorphone hydrochloride solution were given transdermally to patients at a concentration of 8 mg/ml. The experiment started 24 h after operation and lasted for 4 hours. Blood samples were taken at intervals and plasma hydromorphone concentrations were analysed by an HPLC technique. The solution was used in the patients without any untoward effects. The plasma hydromorphone data are shown in Table II below.

Plasma concentrations of hydromorphone in 4 patients postoperatively.

Table II

| Time | Concentration (ng/ml) | | | | Mean±sd |
|---|---|---|---|---|---|
| | Subject | Subject | Subject | Subject | |
| | 1 | 2 | 3 | | |
| Predose | 0 | 1.19* | 0 | 0 | - |
| 1 h | 2.38 | 6.71 | 3.46 | 3.17 | 3.93±1.91 |
| 2 h | 2.59 | 6.48 | 3.63 | 5.66 | 4.59±1.71 |
| 3 h | 3.84 | 6.47 | 3.53 | 5.27 | 4.78±1.36 |
| 4 h | 2.71 | 9.70 | 4.28 | 5.90 | 5.65±3.00 |

* Sample contains interference at the retention time of hydromorphone

Minimum effective analgesic concentration (MEAC) of hydromorphone in plasma is reported to be 4.0 ng/ml (Reidenberg, M, Clin Pharmacol Ther 44, 376-382, 1988.

Example 4

TRANSDERMAL ADMINISTRATION OF MORPHINE ACETATE

Morphine acetate solution was given transdermally in a volunteer (weight 84 Kg). The cellpatch was prefilled with aqueous solution of morphine acetate 100 mg/ml (total dose 100 mg). Blood samples were taken at intervals.
At the moment of application a sting was felt, thereafter the skin site felt similar to intact adjacent skin for the 5 h duration of the experiment. A light erythema was observed transiently. The plasma morphine data are given in Table III below.

Table III

| Time(h) | Morphine(nmol/l) | M6G (nmol/l) | M3G(nmol/l) |
|---|---|---|---|
| 0 | 0 | 0 | 0 |
| 1.0 | 53 | 11.3 | 16.5 |
| 2.0 | 112 | 21.0 | 34.0 |
| 3.0 | 115 | 30.3 | 67.5 |
| 3.5 | 154 | 33.6 | 79.6 |
| 4.0 | 162 | 34.5 | 85.7 |
| 4.5 | 151 | 40.3 | 93.8 |

The above shows plasma concentrations of morphine and its metabolites morphine-6-glucuronide (M6G) and morphine 3-glucuronide (M3G)

Example 5

STABILITY STUDIES

Morphine hydrochloride

Morphine hydrochloride has a maximal solubility of 40 mg/ml in aqueous solution. Long-term stability of solutions containing 20-30 mg morphine per ml may be acceptable for clinical use.

Stability study of morphine hydrochloride solution at 20 mg/ml

Devices in accordance with the invention were filled aseptically in a nitrogen environment. After plugging the drug reservoir, the filled device was sealed in a metallized pocket from which the air was evacuated and replaced with nitrogen gas. The metallized pocket was impermeable to air and light. The samples were stored at 5°C, 25°C, 32°C and 40°C. The following parameters were studied at intervals: appearance, pH, concentrations of morphine hydrochloride trihydrate and pseudomorphine. The samples were also tested for bacterial growth. After 3 months' storage the morphine hydrochloride solution was insignificantly changed and sterile.

Morphine sulphate

Morphine sulphate has a maximal solubility of 45 mg/ml. Long-term stability of morphine sulphate solutions and transdermal utility is not markedly better than with hydrochloride.

Morphine Ascorbate

Ascorbic acid solution (50 mg/ml) dissolves morphine base to 84 mg/ml, yielding a clear solution. The pH of this solution is 5.6. Ascorbic acid $pKa_1$ is 4.17. Ascorbic acid solution at 100 mg/ml dissolves morphine base by same criterion to 166 mg/ml. The pH of this solution is 5.6. The solubility of morphine base in pure water is 0.2 mg/ml. Thus, these results show that the solubility for morphine base in ascorbic acid is determined by the ascorbic acid concentration. Theoretically 1 g of ascorbic acid dissolves 1.62 g anhydrous morphine base and 1.72 g morphine base monohydrate, which corresponds well with the test result described above. An upper limit of solubility of morphine base, with a surplus of ascorbic acid, also exists but was not studied. The solubility for ascorbic acid in pure water is approximately 300 mg/ml.

Long-term stability of solutions of morphine ascorbate can be improved by adding a relative surplus of ascorbate. The pH will then decrease below 5.6. Ascorbic acid solutions are oxydized by air and light and the oxidation is accelerated in the presence of a weak alkali like morphine and transitional metal ions. Storage in an air-free and light-free environment increases long-term stability markedly. Ascorbic acid will also act like an antioxidant to morphine and delay formation of the oxidative degeneration product pseudomorphine.

Morphine ascorbate, biological aspects

Ascorbic acid is a weak acid with suitably high kPa and it is a normal substance in the body and breaks down readily. Together with the qualities of high solubility and antioxidant effect this makes morphine ascorbate an attractive candidate for transdermal delivery.

This solution was tested acutely for transdermal delivery without any untoward effects. At the moment of application a sting was felt, thereafter the site felt completely normal.

Morphine acetate

Morphine acetate is advantageously very water soluble (1:2.5). However, its long-term stability is poor and acetic acid that forms will penetrate through plastic material.

Acetic acid solution (50 mg/ml) readily dissolved morphine base to 100 mg/ml, yielding a clear solution of morphine acetate. NaOH 1M was added, yielding a pH of 5.68. Acetic acid pKa is 4.74.

An upper limit of solubility of morphine base, with a surplus of acetic acid, also exists but was not studied.

Morphine tartrate

The solubility of this salt is approximately 30 mg/ml. Tartaric acid $pKa_1$ is 2.93 and $pKa_2$ 4.23. The binding site configuration helps stabilize morphine salts.

Morphine citrate

Morphine base becomes dissolved to 8 mg/ml in a 0.25m citrate solution at pH 5.6

Example 6

DEVICE COMPRISING FREEZE-DRIED MORPHINE ACETATE

The drug cell of the device was filled with a formulation of 100 mg/ml morphine acetate solution and freeze-dried in place in the cell using a 48h drying cycle. The contents were then dissolved by adding sterile water.

Elsewhere it has also been shown that freeze-drying technology may be applied to morphine acetate, and that such formulations can yield excellent stability over a period of 6 months (Poochkian et al. Morphine acetate. JAMA, 244, 1434, 1980).

**Claims**

1. A transdermal device suitable for continuous administration of an opiate/opioid analgesic for a period of about 24 to 144 hours via an area of skin from which the epidermis has been removed, which device comprises from 11 mg to 3000 mg of morphine or a salt thereof or from $\frac{11}{P}$ to $\frac{3000}{P}$ mg of another opiate/opioid analgesic wherein P is the level of analgesic effect of said other opiate/opioid analgesic relative to morphine.

2. A device as claimed in claim 1 which comprises from 15 mg to 300 mg of morphine or from $\frac{11}{P}$ to $\frac{3000}{P}$ mg of another opiate/opioid analgesic wherein P is as defined in Claim 1.

3. A device as claimed in claim 2 which comprises 100 mg of morphine or $\frac{100}{P}$ mg of another opiate/opioid analgesic wherein P is as defined in Claim 1.

4. A device as claimed in claim 1 or claim 2 wherein the concentration of morphine or other opiate/opioid analgesic is sufficient to deliver a dose of 0.005 - 1.0 mg/kg/hour for a period of 24 to 144 hours.

5.  A device as claimed in any preceding claim wherein said opiate/opioid analgesic is selected from morphine, heroin, hydromorphone, ketobemidone, methadone, oxymorphone, levophanol, alfentanil, fentanyl, meperidine, sufentanil buprenorphine, pentazocine, nalbuphine, butorphanol and the salts thereof.

6.  A device as claimed in claim 5 wherein said opiate/opioid analgesic is selected from the hydrochloride, sulphate, ascorbate, acetate, or tartrate salt of morphine.

7.  A device as claimed in claim 5 or claim 6 which comprises 100mg morphine hydrochloride.

8.  A device as claimed in any preceding claim wherein said opiate/opioid analgesic is present in an aqueous solution.

9.  A device as claimed in one of claims 1 to 8 wherein said opiate/opioid analgesic is present in dried form.

10. A device as claimed in any preceding claim wherein said device also comprises from 1m to 10mg of sodium pyrosulphite.

11. A device as claimed in any preceding claim which further comprises means for separating an area of the epidermis from the dermis and for exposing said area of dermis to said opiate/opioid analgesic.

12. A device as claimed in claim 11 which comprises means to apply suction to the skin to separate the epidermis from the dermis and so form a suction blister.

13. A device as claimed in claim 12 which comprises a blister disruption means.

14. A device as claimed in claim 13 wherein said blister disruption means comprises cutting means operable to sever the roof of the suction blister.

15. A device as claimed in any of claims 11 to 14 which comprises a housing having securing means operable to secure a contact surface of the housing in sealing contact with an area of skin, an aperture defined in the contact surface and communicating with an access port defined by the housing, a suction cup located in the access port, having a lip portion extending peripherally of the aperture and defining an outlet port and a suction chamber which communicates with both the outlet port and the aperture and through which suction may be applied.

16. A device as claimed in claim 15 further comprising cutting means operable to server both the lip portion of the suction cup and the roof of the suction blister once suction has been applied.

17. A device as claimed in claim 15 or claim 16 wherein the outlet port is connectable via a tube to a vacuum source.

18. A device as claimed in claim 17 wherein the connectable vacuum source is an expansion chamber capable of expanding from an initial volume to an expanded volume and further comprising locking means to maintain the expansion chamber in its expanded volume.

19. A device as claimed in claim 18 comprising an indicator responsive to displacement of air along the tube and operable to provide an indication of volumetric displacement of air from the suction chamber in response to formation of a suction blister within the suction chamber.

20. A device as claimed in any one of claims 15 to 19 wherein the housing comprises a base defining the contact surface and a movable portion in which the access port is defined, the movable portion also comprising a reservoir containing said opiate/opioid analgesic, the arrangement being such that after formation of said suction blister and disruption thereof, the movable portion can be moved to bring an outlet of said reservoir into alignment with the aperture in the base of the housing and hence the exposed area of dermis.

21. A device as claimed in claim 20 wherein the movable portion is rotatable and the access port and the outlet of the reservoir are both at an eccentric location relative to the axis of rotation.

22. A device as claimed in any preceding claim wherein said opiate/opioid analgesic is in a suspension, a saturated solution, a supersaturated solution or in a dried form.

**23.** A device as claimed in any one of claims 9 to 21 which is adapted for co-operation with means to supply a liquid for reconstitution of said dried opiate/opioid analgesic.

**24.** A device as claimed in any one of claims 9 to 21 which includes therein a reservoir containing a liquid for reconstitution of said dried opiate/opioid analgesic and means for breaking or disrupting said reservoir to bring said liquid into contact with the dried opiate/opioid analgesic.

**25.** A device as claimed in any preceding claim wherein said opiate/opioid analgesic is contained in a porous matrix of a polymeric material.

**26.** A device as claimed in any one of claims 1 to 10 which is of a flexible laminar structure in which said opiate/opioid analgesic is dispersed or otherwise impregnated in a layer comprising a porous matrix of a polymeric material.

**27.** A device as claimed in claim 26 wherein the porous matrix of a polymeric material acts as an adhesive to secure said device to the skin.

**28.** A device as claimed in any one of claims 1 to 10 which comprises means for separating an area of the epidermis from the dermis to form a suction blister, means for severing the roof of said suction blister to expose an area of de-epithelialized skin and means for applying a patch to said exposed area of de-epithelialized skin wherein said patch has said opiate/opioid analgesic dispersed or otherwise impregnated therein.

**Patentansprüche**

**1.** Transdermale Vorrichtung, die sich zur kontinuierlichen Verabreichung eines Opiats / opioiden Analgetikums über einen Zeitraum von 24 bis 144 Stunden durch einen Hautbereich eignet, von dem die Epidermis entfernt worden ist, und welche von 11 mg bis 3000 mg Morphin enthält oder eines Salzes davon oder zwischen 11/P bis 3000/P mg eines anderen Opiats / opioiden Analgetikums, wobei P der Grad des analgetischen Effekts des besagten anderen Opiats / opioiden Analgetikums gegenüber Morphin ist.

**2.** Vorrichtung nach Anspruch 1, welche zwischen 15 mg und 300 mg Morphin enthält oder von 15/P bis 300/P mg eines anderen Opiats / opioiden Analgetikums, wobei P wie in Anspruch 1 definiert ist.

**3.** Vorrichtung nach Anspruch 2, welche 100 mg Morphin enthält oder 100/P mg eines anderen Opiats / opioiden Analgetikums, wobei P wie in Anspruch 1 definiert ist.

**4.** Vorrichtung nach Anspruch 1 oder 2, wobei die Konzentration des Morphins oder des anderen Opiats / opioiden Analgetikums ausreichend ist, um eine Dosis von 0,005 - 1,0 mg/kg/Stunde über einen Zeitraum von 24 bis 144 Stunden hinweg abzugeben.

**5.** Vorrichtung nach einem der vorhergehenden Ansprüche, wobei das besagte Opiat / opioide Analgetikum ausgewählt ist aus Morphin, Heroin, Hydromorphon, Ketobemidon, Methadon, Oxymorphon, Levorphanol, Alfentanil, Phentanyl, Meperidin, Sufentanil-Buprenorphin, Pentazocin, Nalbuphin, Butorphanol und deren Salzen.

**6.** Vorrichtung nach Anspruch 5, wobei das besagte Opiat / opioide Analgetikum ausgewählt ist aus einem salzsauren Salz, Sulfat, Ascorbat, Acetat, oder einem Weinsäuresalz von Morphin.

**7.** Vorrichtung nach Anspruch 5 oder 6, welche 100 mg Morphin-Hydrochlorid enthält.

**8.** Vorrichtung nach einem der vorhergehenden Ansprüche, wobei das besagte Opiat / opioide Analgetikum in einer wäßrigen Lösung vorliegt.

**9.** Vorrichtung nach einem der Ansprüche 1 bis 8, wobei das besagte Opiat / opioide Analgetikum in getrockneter Form vorliegt.

**10.** Vorrichtung nach einem der vorhergehenden Ansprüche, welche auch von 1 mg bis 10 mg Natriumdisulfit enthält.

**11.** Vorrichtung nach einem der vorhergehenden Ansprüche, welche ferner ein Mittel aufweist zum Trennen eines Bereichs der Epidermis von der Dermis, und um den besagten Bereich der Dermis dem besagten Opiat / opioiden Analgetikum auszusetzen.

**12.** Vorrichtung nach Anspruch 11, welche ferner ein Mittel aufweist, um die Haut einem Unterdruck auszusetzen zum Zwecke des Abtrennens der Epidermis von der Dermis und der Formung einer Saugblase.

**13.** Vorrichtung nach Anspruch 12, welche ein Mittel zum Aufreißen einer Blase aufweist.

**14.** Vorrichtung nach Anspruch 13, wobei das besagte Mittel zum Aufreißen einer Blase ein Schneidmittel aufweist, das betätigbar ist, um die Decke der Saugblase abzutrennen.

**15.** Vorrichtung nach einem der Ansprüche 11 bis 14, welche ein Gehäuse aufweist mit Fixierungsmitteln, welche betätigbar sind, um eine Kontaktoberfläche des Gehäuses in abgedichteten Kontakt mit einem Hautbereich zu bringen, eine von der Kontaktoberfläche umgrenzte Öffnung, die mit einem von dem Gehäuse gebildeten Zulaufanschluß kommuniziert, einen in dem Zulaufanschluß angeordneten Saugbecher mit einem Lippenbereich, der sich peripher zu der Öffnung erstreckt und einen Auslaßanschluß und eine Saugkammer umschließt, die sowohl mit dem Auslaßanschluß als auch mit der Öffnung kommuniziert, und durch welche ein Unterdruck angelegt werden kann.

**16.** Vorrichtung nach Anspruch 15, welche ferner ein Schneidmittel aufweist, das betätigbar ist, um sowohl den Lippenbereich des Saugbechers als auch die Decke der Saugblase abzutrennen, nachdem der Unterdruck angelegt worden ist.

**17.** Vorrichtung nach Anspruch 15 oder Anspruch 16, wobei der Auslaßanschluß über eine Rohrleitung an eine Vakuumquelle anschließbar ist.

**18.** Vorrichtung nach Anspruch 17, wobei die anschließbare Vakuumquelle als Expansionskammer ausgebildet ist, welche in der Lage ist, sich von einem anfänglichen Volumen auf ein expandiertes Volumen auszudehnen, und weiterhin ein Verschlußmittel aufweist, um die Expansionskammer in ihrem expandierten Volumen zu halten.

**19.** Vorrichtung nach Anspruch 18, welche ein Indikationsmittel aufweist, das auf Luftverschiebungen entlang der Leitung reagiert und betätigbar ist, um eine Anzeige der volumetrischen Luftverschiebung von der Saugkammer zu bieten als Reaktion auf die Bildung einer Saugblase innerhalb der Saugkammer.

**20.** Vorrichtung nach einem der Ansprüche 15 bis 19, wobei das Gehäuse eine Basis umfaßt, welche eine Kontaktoberfläche bildet, und einen beweglichen Bereich, von welchem der Zulaufanschluß umschlossen wird, wobei der bewegliche Bereich auch ein Reservoir aufweist, welches das besagte Opiat / opioide Analgetikum enthält, und wobei die Anordnung derart getroffen ist, dass nach der Bildung einer Saugblase und dem Aufreißen derselben der bewegliche Bereich bewegt werden kann, um einen Auslaß des besagten Reservoirs in eine Flucht mit der Öffnung in der Basis des Gehäuses zu bringen und weiter mit dem entblößten Bereich der Dermis.

**21.** Vorrichtung nach Anspruch 20, wobei der bewegliche Bereich rotierbar ist und sich der Zulaufanschluß wie auch der Auslaßanschluß des Reservoirs jeweils an einer exzentrischen Position im Verhältnis zu der Rotationsachse befindet.

**22.** Vorrichtung nach einem der vorhergehenden Ansprüche, wobei das besagte Opiat / opioide Analgetikum in einer Suspension vorliegt, einer gesättigten Lösung, einer übersättigten Lösung oder in einer getrockneten Form.

**23.** Vorrichtung nach einem der Ansprüche 9 bis 21, welche für ein Zusammenwirken mit einem Mittel zum Zuführen einer Flüssigkeit zwecks rückbildenden Umformung eines getrockneten Opiats / opioiden Analgetikums.

**24.** Vorrichtung nach einem der Ansprüche 9 bis 21, welche ein Reservoir umfaßt, welches eine Flüssigkeit enthält zur rückbildenden Umformung eines getrockneten Opiats / opioiden Analgetikums, und ein Mittel zum Brechen oder Aufreißen des besagten Reservoirs, um die Flüssigkeit in Kontakt mit dem getrockneten Opiat / opioiden Analgetikum zu bringen.

**25.** Vorrichtung nach einem der vorhergehenden Ansprüche, wobei das besagte Opiat / opioide Analgetikum in einer porösen Matrix eines polymeren Materials enthalten ist.

**26.** Vorrichtung nach einem der Ansprüche 1 bis 10, welche von flexibler, laminarer Struktur ist, bei welcher das besagte Opiat / opioide Analgetikum in einer Schicht verteilt oder anderweitig aufgenommen ist, welche aus einer porösen Matrix eines polymeren Materials gebildet ist.

**27.** Vorrichtung nach Anspruch 26, wobei die poröse Matrix eines polymeren Materials als Klebemittel wirkt, um die Vorrichtung an der Haut festzulegen.

**28.** Vorrichtung nach einem der Ansprüche 1 bis 10, welche ein Mittel zum Trennen eines Bereichs der Epidermis von der Dermis aufweist, um eine Saugblase zu bilden, ferner ein Mittel zum Abtrennen der Decke der besagten Saugblase, um einen Bereich der de-epithelisierten Haut bloßzulegen, und schließlich ein Mittel zum Aufbringen eines Pflasters auf den besagten, entblößten Bereich der de-epithelisierten Haut, wobei das besagte Pflaster das besagte Opiat / opioide Analgetikum in verteilter Form enthält oder damit getränkt ist.

**Revendications**

**1.** Dispositif transdermique apte à l'administration continue d'un analgésique opiacé/opioïde pendant une période de temps d'environ 24 à 144 heures par une zone cutanée de laquelle l'épiderme est éliminé, dispositif qui comprend 11 mg à 3000 mg de morphine ou d'un de ses sels ou à $\frac{3000}{P}$ mg d'un autre analgésique opiacé/opioïde, P étant le degré d'effet analgésique dudit autre analgésique opiacé/opioïde par rapport à la morphine.

**2.** Dispositif suivant la revendication 1, qui comprend 15 mg à 300 mg de morphine ou $\frac{15}{P}$ à $\frac{300}{P}$ mg d'un autre analgésique opiacé/opioïde, P répondant à la définition figurant dans la revendication 1.

**3.** Dispositif suivant la revendication 2, qui comprend 100 mg de morphine ou $\frac{100}{P}$ mg d'un autre analgésique opiacé/opioïde, P répondant à la définition figurant dans la revendication 1.

**4.** Dispositif suivant la revendication 1 ou la revendication 2, dans lequel la concentration de morphine ou d'un autre analgésique opiacé/opioïde est suffisante pour délivrer une dose de 0,005 à 1,0 mg/kg/heure pendant une période de temps de 24 à 144 heures.

**5.** Dispositif suivant l'une quelconque des revendications précédentes, dans lequel ledit analgésique opiacé/opioïde est choisi entre la morphine, l'héroïne, l'hydromorphone la kétobémidone, la méthadone, l'oxymorphone, le lévophanol, l'alfentanil, le fentanyle, la mépéridine, le sufentanil, la buprénorphine, la pentazocine, la nalbuphine, le butorphanol et leurs sels.

**6.** Dispositif suivant la revendication 5, dans lequel ledit analgésique opiacé/opioïde est choisi parmi les sels consistant en chlorhydrate, sulfate, ascorbate, acétate et tartrate de morphine.

**7.** Dispositif suivant la revendication 5 ou la revendication 6, qui comprend 100 mg de chlorhydrate de morphine.

**8.** Dispositif suivant l'une quelconque des revendications précédentes, dans lequel ledit analgésique opiacé/opioïde est présent dans une solution aqueuse.

**9.** Dispositif suivant une des revendications 1 à 8, dans lequel ledit analgésique opiacé/opioïde est présent sous forme déshydratée.

**10.** Dispositif suivant l'une quelconque des revendications précédentes, ledit dispositif comprenant également 1 mg à 10 mg de pyrosulfite de sodium.

**11.** Dispositif suivant l'une quelconque des revendications précédentes, qui comprend en outre un moyen pour séparer une zone de l'épiderme du derme et pour exposer ladite zone du derme audit analgésique opiacé/opioïde.

**12.** Dispositif suivant la revendication 11, qui comprend un moyen pour soumettre la peau à une aspiration pour séparer l'épiderme du derme et former ainsi une cloque d'aspiration.

**13.** Dispositif suivant la revendication 12, qui comprend un moyen de rupture de cloque.

**14.** Dispositif suivant la revendication 13, dans lequel ledit moyen de rupture de cloque comprend un moyen de coupe pouvant fonctionner pour sectionner la racine de la cloque d'aspiration.

**15.** Dispositif suivant l'une quelconque des revendications 11 à 14, qui comprend un boîtier comprenant un moyen de fixation capable de fixer une surface de contact du boîtier en contact hermétique avec une zone cutanée, une ouverture définie dans la surface de contact et communiquant avec un orifice d'accès défini par le boîtier, une ventouse située dans l'orifice d'accès, comportant une lèvre s'étendant à la périphérie de l'ouverture et définissant un orifice de sortie et une chambre d'aspiration qui communique à la fois avec l'orifice de sortie et l'ouverture et à travers laquelle une aspiration peut être effectuée.

**16.** Dispositif suivant la revendication 15, comprenant en outre un moyen de coupe pouvant fonctionner pour sectionner à la fois la lèvre de la ventouse et la racine de la cloque d'aspiration, une fois l'aspiration effectuée.

**17.** Dispositif suivant la revendication 15 ou la revendication 16, dans lequel l'orifice de sortie peut être connecté par un tube à une source de vide.

**18.** Dispositif suivant la revendication 17, dans lequel la source de vide pouvant être connectée est une chambre d'expansion apte à l'expansion d'un volume initial à un volume à l'état expansé et comprenant en outre un moyen de verrouillage pour maintenir la chambre d'expansion au volume expansé.

**19.** Dispositif suivant la revendication 18, comprenant un indicateur sensible au déplacement d'air le long du tube et pouvant fonctionner pour fournir une indication de déplacement volumétrique d'air à partir de la chambre d'aspiration en réponse à la formation d'une cloque d'aspiration à l'intérieur de la chambre d'aspiration.

**20.** Dispositif suivant l'une quelconque des revendications 15 à 19, dans lequel le boîtier comprend une base définissant la surface de contact et une partie mobile dans laquelle l'orifice d'accès est défini, la partie mobile comprenant également un réservoir contenant ledit analgésique opiacé/opioïde, la configuration étant telle que, après formation de ladite cloque d'aspiration et sa rupture, la partie mobile puisse être déplacée pour mettre un orifice de sortie dudit réservoir en alignement avec l'ouverture dans la base du boîtier et donc la zone exposée du derme.

**21.** Dispositif suivant la revendication 20, dans lequel la partie mobile est rotative et l'orifice d'accès et l'orifice de sortie du réservoir sont tous deux à un emplacement excentrique par rapport à l'axe de rotation.

**22.** Dispositif suivant l'une quelconque des revendications précédentes, dans lequel ledit analgésique opiacé/opioïde est présent dans une suspension, une solution saturée, une solution sursaturée ou bien est sous forme déshydratée.

**23.** Dispositif suivant l'une quelconque des revendications 9 à 21, qui est adapté à la coopération avec un moyen pour fournir un liquide pour la reconstitution dudit analgésique opiacé/opioïde déshydraté.

**24.** Dispositif suivant l'une quelconque des revendications 9 à 21, qui renferme un réservoir contenant un liquide pour la reconstitution dudit analgésique opiacé/opioïde déshydraté et un moyen pour rompre ou briser ledit réservoir afin de mettre ledit liquide en contact avec l'analgésique opiacé/opioïde déshydraté.

**25.** Dispositif suivant l'une quelconque des revendications précédentes, dans lequel ledit analgésique opiacé/opioïde est présent dans une matrice poreuse d'une matière polymère.

**26.** Dispositif suivant l'une quelconque des revendications 1 à 10, qui a une structure laminaire flexible dans laquelle ledit analgésique opiacé/opioïde est dispersé dans, ou bien imprègne d'une autre manière, une couche comprenant une matrice poreuse d'une matière polymère.

**27.** Dispositif suivant la revendication 26, dans lequel la matrice poreuse d'une matière polymère joue le rôle d'adhésif pour fixer ledit dispositif à la peau.

**28.** Dispositif suivant l'une quelconque des revendications 1 à 10, qui comprend un moyen pour séparer une zone de l'épiderme du derme afin de former une cloque d'aspiration, un moyen pour sectionner la racine de ladite cloque d'aspiration afin d'exposer une zone de peau de-épithélialisée et un moyen pour appliquer un timbre à ladite zone exposée de peau de-épithélialisée, ledit timbre comprenant ledit analgésique opiacé/opioïde dispersé dans ce timbre ou bien l'imprégnant d'une autre manière.

FIG. 1.

FIG. 2.

FIG. 3.

FIG. 4.

FIG. 5.

FIG. 6a.

FIG. 6.

FIG. 7.

FIG. 8.

FIG. 9.

FIG. 10.

FIG. 11.

FIG. 12.

FIG. 13.

FIG. 14.

FIG. 15.

FIG. 16.

FIG. 17.